# DEMANDE DE BREVET EUROPEEN

(11) **EP 1 714 639 A1**
(43) Date de publication de la demande: **25.10.2006**
(21) Numéro de dépôt: 06111879.0
(22) Date de dépôt: 29.03.2006
(51) Int. Cl.: A61K 8/49, A61Q 19/08

(54) **Compositions cosmétiques comprenant des dérivés de pipérazine**

(30) Priorité: 21.04.2005 FR 0551025
(71) Demandeur: L'ORÉAL, 75008 Paris (FR)
(72) Inventeur: Cavezza, Alexandre, 93320 Pavillon sous Bois (FR)
(74) Mandataire: Kromer, Christophe

(57) **Abrégé**

La présente invention concerne une composition comprenant un milieu physiologiquement acceptable adapté à une application topique sur la peau, qui contient au moins un dérivé de pipérazine de formule :

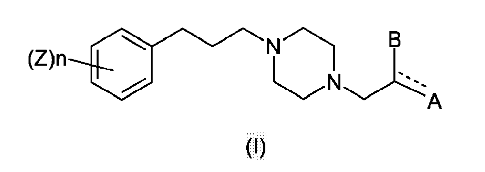

Elle concerne également un procédé de traitement cosmétique d'une peau ridée, comprenant l'application topique sur la peau de cette composition, ainsi que deux nouvelles familles de dérivés de pipérazine.

## Description

La présente invention concerne une composition comprenant un milieu physiologiquement acceptable adapté à une application topique sur la peau, qui contient au moins un dérivé de pipérazine de formule donnée. Elle concerne également un procédé de traitement cosmétique d'une peau ridée, comprenant l'application topique sur la peau de cette composition, ainsi que deux nouvelles familles de dérivés de pipérazine.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en améliorant son renouvellement cellulaire ou encore en favorisant la synthèse, ou en prévenant la dégradation des fibres élastiques qui composent le tissu cutané.

Bien que ces traitements permettent d'agir sur les rides et ridules dues au vieillissement chronologique ou intrinsèque, ainsi que sur celles dues au photo-vieillissement, ils n'ont pas d'effet sur les rides d'expression, lesquelles nécessitent une intervention sur la composante contractile musculaire (via des agents myorelaxants) ou dermique (via des agents dermo-décontractant) des rides.

Les rides d'expression sont en effet la résultante de mécanismes différents de ceux générant les rides dues au vieillissement.

Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.

Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, le seul moyen couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21).

La Demanderesse a en outre proposé divers composés susceptibles d'offrir un effet myorelaxant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques, tels que le vérapamil (FR-2 793 681), et en particulier le manganèse et ses sels (FR-2 809 005) et l'alvérine (FR-2 798 590) ; les agonistes des récepteurs associés aux canaux chlore, dont la glycine (EP-0 704 210) et certains extraits d'*Iris pallida* (FR-2 746 641) ; et les sapogénines (EP-1 352 643).

A côté de ces agents myorelaxants, la Demanderesse a décrit divers composés dermo-décontractants et en particulier l'adénosine (EP-1 424 064) et des composés aminés (EP-1 405 633).

Il reste toutefois le besoin de disposer d'autres composés efficaces pour lisser ou estomper les rides, en particulier d'expression.

En particulier, le but de la présente invention est de proposer des composés ayant des propriétés myorelaxantes tout en offrant une biodisponibilité améliorée par rapport à d'autres agents dermo-décontractants ou myorelaxants tels que les inhibiteurs calciques connus.

Or la Demanderesse a découvert avec étonnement que certains dérivés de pipérazine permettaient de satisfaire ce besoin.

La présente invention a donc pour objet une composition comprenant un milieu physiologiquement acceptable adapté à une application topique sur la peau, qui contient au moins un dérivé de pipérazine choisi parmi les composés de formule (I) : 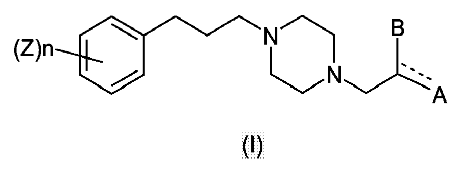 dans laquelle :
- chacun des groupes Z désigne indépendamment un atome de fluor, un radical alkyle linéaire en C₁-C₆ ou ramifié en C₃-C₆, un radical alkényle linéaire en C₂-C₆ ou ramifié en C₃-C₆, un radical cycloalkyle en C₃-C₆, un groupe CF₃, un groupe OR₁, un groupe NR₁R₂, un groupe COR₁, un groupe COOR₁, un groupe CONR₁R₂ ou un groupe N(R₁)COR₂ ;
- n va de 0 à 5 ;
- A désigne :

- un atome d'hydrogène,
- un radical alkyle linéaire en C₁-C₂₀ ou ramifié en C₃-C₂₀ ,
- un radical alkényle en C₂-C₂₀ linéaire ou en C₃-C₂₀ ramifié,
- un radical cycloalkyle en C₃-C₂₀,
   lesdits radicaux étant éventuellement substitués par au moins un groupe choisi parmi : un radical oxo, un groupe OR₁, un groupe NR₁R₂, un groupe COR₁, un groupe COOR₁, un groupe CONR₁R₂, un groupe N(R₁)COR₂, ou un radical phényle éventuellement substitué par un à cinq groupes Z tels que définis ci-dessus,
- un radical phényle éventuellement substitué par un à cinq groupes Z tels que définis ci-dessus
- B désigne un atome d'hydrogène, un radical alkyle linéaire en C₁-C₆ ou ramifié en C₃₋C₆, un radical alkényle linéaire en C₂-C₆ ou ramifié en C₃-C₆, un radical cycloalkyle en C₃-C₆, un groupe OR₁, un groupe NR₁R₂, un groupe COR₁, un groupe COOR₁, un groupe CONR₁R₂ ou un groupe N(R₁)COR₂ ;
où R₁ et R₂ désignent indépendamment un atome d'hydrogène, un radical alkyle linéaire en C₁-C₆ ou ramifié en C₃-C₆ éventuellement substitué par un radical phényle, un radical alkényle linéaire en C₂-C₆ ou ramifié en C₃-C₆, un radical cycloalkyle en C₃₋C₆ ou un radical phényle ; les groupes R₁, respectivement R₂, pouvant être identiques
ou différents les uns des autres ;
les pointillés désignent une double liaison potentielle, étant entendu que A est distinct d'un atome d'hydrogène ou d'un radical phényle lorsque cette double liaison est présente,
et leurs sels, isomères optiques et solvates.

L'invention a aussi pour objet l'utilisation cosmétique de la composition précitée pour le soin de la peau, en particulier pour le soin de la peau ridée et/ou pour lisser les rides, détendre les traits et/ou décontracter la peau.

Elle a aussi pour objet l'utilisation cosmétique d'au moins un dérivé de pipérazine tel que défini ci-dessus, comme agent pour atténuer les rides et/ou décontracter la peau et/ou détendre les traits.

Elle a encore pour objet un procédé de traitement cosmétique d'une peau ridée, en particulier la peau du visage et/ou du front, comprenant l'application topique sur ladite peau d'une composition telle que définie précédemment.

Au sens de la présente invention, on entend en particulier par "rides" les rides présentes sur le visage, y compris le front, et dans le cou. Il s'agit plus particulièrement de rides d'expression, dues aux mimiques répétées et accentuées par l'âge. Les rides concernées sont de préférence celles disposées radialement autour de la bouche et/ou des yeux, en particulier les rides de la patte d'oie, et/ou situées au niveau du front, en particulier la ride dite du lion, située au niveau de la glabelle, dans l'espace inter-sourcillier, et/ou disposées horizontalement sur le front.

La composition selon l'invention est avantageusement destinée à être appliquée sur les zones du visage et/ou du front marquées par des rides d'expression, et/ou sur les personnes présentant des rides d'expression.

Par ailleurs, par "milieu physiologiquement acceptable", on entend un milieu compatible avec la peau et plus particulièrement avec la peau du visage et/ou du cou. Ce milieu est avantageusement cosmétiquement acceptable, c'est-à-dire qu'il n'entraîne pas de démangeaisons, de picotements ou de rougeurs inacceptables, susceptibles de détourner l'utilisateur de la composition, et qu'il présente un aspect, une odeur et un toucher agréables.

Dans la formule (I), les groupes alkyle peuvent notamment être choisis, selon le cas, parmi les groupes : méthyle, éthyle, n-propyle, isopropyle, n-butyle, isobutyle, tert-butyle, pentyle, hexyle, heptyle, octyle, nonyle, décyle, undécyle, dodécyle, myristyle, et palmityle.

En outre, dans le contexte de cette demande, on entend par "alkenyle" des radicaux pouvant comprendre une ou plusieurs doubles liaisons, conjuguées ou non. Ils peuvent notamment être choisis, selon le cas, parmi les groupes : vinyle, allyle, butényle ou pentényle.

Enfin, des exemples de radicaux cycloalkyle sont les radicaux cyclobutyle, cyclopentyle et cyclohexyle.

Par "substitués", on entend des radicaux, en particulier alkyle, qui peuvent être substitués par des groupements latéraux ou par des groupements situés à l'extrémité de la chaîne desdits radicaux. Ainsi, on entend notamment par "radical alkyle substitué par un radical phényle" des radicaux autrement désignés par "aralkyle".

Comme sels du composé de formule (I), on peut citer les sels obtenus par addition du composé de formule (I) avec un acide minéral, choisi notamment parmi les acides chlorhydrique, sulfurique et phosphorique, ou avec un acide organique, choisi en particulier parmi les acides acétique, propionique, succinique, fumarique, lactique, glycolique, citrique et tartrique.

De préférence, le dérivé de pipérazine selon l'invention est tel que lorsque B est un atome d'hydrogène, alors A est distinct d'un groupe phényle éventuellement substitué.

En outre, selon une forme d'exécution avantageuse de l'invention, chacun des groupes Z désigne indépendamment un groupe choisi parmi : un atome de fluor, un groupe OR₁, un groupe NR₁R₂, un groupe N(R₁)COR₂, un groupe COOR₁ ou un groupe CF₃. Plus préférentiellement, Z désigne un groupe OR₁ ou un groupe COOR₁, de préférence un groupe OR₁. Selon une forme d'exécution préférée, R₁ et R₂ désignent alors indépendamment : un atome d'hydrogène ou un radical alkyle linéaire en C₁-C₆ ou ramifié en C₃-C₆, de préférence un radical méthyle ou éthyle.

En variante, le dérivé de pipérazine selon l'invention est tel que n = 0.

En outre, selon un mode de réalisation avantageux de l'invention, A désigne :
- un atome d'hydrogène,
- un radical alkyle linéaire en C₁-C₈ ou ramifié en C₃-C₈,
- un radical alkényle linéaire en C₂-C₈ ou ramifié en C₃-C₈,
- un radical cycloalkyle en C₃-C₈,
   lesdits radicaux étant éventuellement substitués par au moins un groupe choisi parmi : un radical oxo, un groupe OR₁, un groupe NR₁R₂, ou un radical phényle éventuellement substitué par un à cinq groupes Z tels que définis dans la formule (I), ou
- un radical phényle éventuellement substitué par un à cinq groupes Z tels que définis dans la formule (I).

Mieux, on préfère que A désigne un atome d'hydrogène ou un radical alkyle linéaire en C₁-C₆ ou ramifié en C₃-C₆, éventuellement substitué par au moins un groupe choisi parmi : un radical oxo, un groupe OR₁, ou un radical phényle, où R₁ désigne de préférence un atome d'hydrogène ou peut désigner un radical alkyle linéaire en C₁-C₆ ou ramifié en C₃-C₆ tel qu'un radical méthyle.

Par ailleurs, les dérivés de pipérazine préférés selon l'invention sont tels que B désigne un atome d'hydrogène ; un radical alkyle en C₁-C₆ linéaire ou en C₃-C₆ ramifié ; un groupe NR₁R₂ ; un groupe COR₁ ; ou un groupe OR1, où R₁ désigne un atome d'hydrogène, un radical alkyle en C₁-C₆, linéaire ou en C₃-C₆ ramifié, éventuellement substitué par un radical phényle.

Mieux, B désigne un atome d'hydrogène, un groupe OR₁ ou un groupe COR₁ où R₁ désigne un radical alkyle linéaire en C₁-C₆ linéaire ou ramifié en C₃-C₆, éventuellement substitué par un radical phényle.

Parmi les composés de formule (I) décrits précédemment, on utilise de préférence ceux pour lesquels :
- A désigne un radical alkyle linéaire en C₁-C₈ éventuellement substitué par un radical phényle, B désigne un atome d'hydrogène ou un groupe hydroxyle, et n = 0 ;
ou
- A désigne un atome d'hydrogène, B désigne -OR₁ ou -COR₁ avec R₁ désignant un radical alkyle linéaire en C₁-C₆ éventuellement substitué par un radical phényle et n = 0.

Préférentiellement, on utilise les composés de formule (I) pour lesquels :
- A désigne un radical alkyle linéaire en C₁-C₈ éventuellement substitué par un radical phényle, B désigne un atome d'hydrogène et n = 0 ;
ou
- A désigne un atome d'hydrogène, B désigne -OR₁ avec R₁ désignant un radical alkyle linéaire en C₁-C₆ substitué par un radical phényle et n = 0 ;
ou
- A désigne un radical alkyle en C₁-C₆ , B désigne -OH et n = 0 ;
ou
- A désigne un atome d'hydrogène, B désigne -COR₁ avec R₁ désignant un radical alkyle linéaire en C₁-C₆ et n = 0.

Des dérivés de pipérazine préférés pour une utilisation dans la présente invention sont listés dans le Tableau 1 ci-après. Ce Tableau indique également les valeurs de poids moléculaire et de cLogP des composés selon l'invention. La valeur de cLogP peut être calculée comme décrit dans le logiciel Advanced Chemistry Development (ACD/Labs) Software Solaris V4.67.

Dans le cadre de ses recherches sur la biodisponibilité des composés cosmétiques, la Demanderesse a en effet démontré que pour traverser le stratum corneum, qui est la couche superficielle de la peau, les composés cosmétiques et en particulier des composés ionisables comme les pipérazines devaient avoir un poids moléculaire inférieur à 400, et de préférence un poids moléculaire inférieur à 350, et/ou une valeur de cLogP (coefficient de partage octanol / eau calculé de préférence inférieur à 5 et si possible inférieur à 4.

Comme il ressort du Tableau 1, les composés selon l'invention ont tous un poids moléculaire inférieur à 400 et/ou une valeur de cLogP inférieur à 5.

Par comparaison, la cinnarizine et la flunarizine, qui sont deux anti-calciques de référence ayant une structure proche des composés selon l'invention, ont respectivement une valeur de cLogP de 6.0 et 6.3.

Ainsi, les composés selon l'invention ont une meilleure biodisponibilité que les agents anti-calciques connus de structure proche.

**TABLEAU 1**

| **Exemples de dérivés de pipérazine selon l'invention** | | | | |
|---|---|---|---|---|
| Ex. | RN | NOM | clogP | PM |
| 1 | 048203-82-3 | 1-(3-phenyl-2-propenyl)-4-(3-phenylpropyl)- piperazine | 4,5 | 320 |
| 2 | 056233-37-5 | 1-(2-phenylethyl)-4-(3-phenylpropyl)- piperazine | 4,0 | 308 |
| 3 | 067469-80-1 | 1-(2-hydroxyéthyl) 4-(3-phenylpropyl)-piperazine | 1,7 | 248 |
| 4 | 067514-13-0 | 1-(3-hydroxpropyl) 4-(3-phenylpropyl)-piperazine | 1,6 | 262 |
| 5 | 076087-26-8 | 1-ethyl 4-(3-phenylpropyl)- piperazine | 2,5 | 232 |
| 6 | 076087-28-0 | 1-propyl 4-(3-phenylpropyl)-piperazine | 3,1 | 246 |
| 7 | 076087-32-6 | 1-butyl-4-(3-phenylpropyl)-piperazine | 3,6 | 260 |
| 8 | 076087-34-8 | 1-(2-methylpropyl) 4-(3-phenylpropyl)-piperazine | 3,4 | 260 |
| 9 | 076087-36-0 | 1-pentyl 4-(3-phenylpropyl)-piperazine | 4,1 | 274 |
| 10 | 076087-38-2 | 1-hexyl4-(3-phenylpropyl)-piperazine | 4,7 | 288 |
| 11 | 076087-40-6 | 1-heptyl4-(3-phenylpropyl)-piperazine | 5,2 | 302 |
| 12 | 076087-44-0 | 1-(3-phenylpropyl) 4-(2-propenyl)-piperazine | 2,9 | 244 |
| 13 | 076087-46-2 | 1-(2-butenyl) 4-(3-phenylpropyl)-piperazine | 3,4 | 258 |
| 14 | 076087-54-2 | 1,4-bis-(3-phenylpropyl)-piperazine | 4,6 | 322 |
| 15 | 076087-68-8 | 1-[N,N-diethyl (2-aminoéthyl)] 4-(3-phenylpropyl)-piperazine | 3,2 | 303 |
| 16 | 082075-48-7 | 1-(3-phénylpropan-3-one)-4-(3-phenylpropyl)-pipérazine | 3,7 | 336 |
| 17 | 086621-39-8 | 4-[2-[4-(3-phenylpropyl)-1-piperazinyl]ethyl]-benzoate d'éthyle | 4,5 | 380 |
| 18 | 086621-53-6 | acide 4-[2-[4-(3-phenylpropyl)-1-piperazinyl]ethyl] benzoïque | 3,6 | 352 |
| 19 | 165377-43-5 | 1-[2-(3,4-dimethoxyphenyl)ethyl] 4-(3-phenylpropyl) piperazine | 3,7 | 368 |
| 20 | 197451-51-7 | 1-[2-(3,4-dimethoxyphenyl)ethyl] 4-[3-(4-fluorophenyl)propyl] piperazine | 3,8 | 386 |
| 21 | 200885-91-2 | 1-(2-phenylpropyl)-4-(3-phenylpropyl) piperazine | 4,3 | 322 |
| 22 | 219659-23-1 | 2-[2-[4-(3-phenylpropyl)-1-piperazinyl]ethyl] benzenamine | 2,7 | 323 |
| 23 | 219659-24-2 | 3-[2-[4-(3-phenylpropyl)-1-piperazinyl]ethyl] benzenamine | 2,7 | 323 |
| 24 | 219659-25-3 | 4-[2-[4-(3-phenylpropyl)-1-piperazinyl]ethyl] benzenamine | 2,7 | 323 |
| 25 | 219659-26-4 | 1-[2-(2-methoxyphenyl)ethyl] 4-(3-phenylpropyl) piperazine | 3,9 | 338 |
| 26 | 219659-27-5 | 1-[2-(3-methoxyphenyl)ethyl]-4-(3-phenylpropyl) piperazine | 3,9 | 338 |
| 27 | 219659-28-6 | 1-[2-(4-methoxyphenyl)ethyl]-4-(3-phenylpropyl) piperazine | 3,9 | 338 |
| 28 | 219659-40-2 | 1-(2-aminoéthyl)-4-(3-phenylpropyl)-piperazine | 1,3 | 247 |
| 29 | 219659-41-3 | 1-(3-aminopropyl) 4-(3-phenylpropyl)-piperazine | 1,7 | 261 |
| 30 | 219659-42-4 | 1-(4-aminobutyl)-4-(3-phenylpropyl)-piperazine | 2,0 | 275 |
| 31 | 422575-11-9 | N-méthyl, N-(2-phenylethyl)-1- (3-aminopropyl)- 4-(3-phenylpropyl)-piperazine | 4,5 | 380 |
| 32 | 425644-73-1 | 2-methoxy-4-[2-[4-(3-phenylpropyl)-1-piperazinyl]ethyl]-phénol | 2,9 | 354 |
| 33 | 652159-28-9 | 2-methoxy 4-[2-[4-[3-(4-fluorophenyl)propyl]-1-piperazinyl]ethyl] phénol | 3,0 | 372 |
| 34 | 652159-29-0 | 1-[2-(4-ethoxy-3-methoxyphenyl)ethyl]-4-(3-phenylpropyl) piperazine | 4,2 | 382 |
| 35 | 652159-30-3 | 1-[2-(4-ethoxy-3-methoxyphenyl)ethyl]-4-[3-(4-fluorophenyl)propyl] piperazine | 4,3 | 400 |
| 36 | 690200-18-1 | 1-[4-(4-fluorophényl)-butan-4-one]-4-[3-(4-méthoxyphényl)-propyl]-piperazine | 4,1 | 398 |
| 37 | 703397-45-9 | 1-(3-hydroxy 2-phenyl propyl) 4-(3-phenylpropyl) piperazine | 3,3 | 338 |
| 38 | 712261-46-6 | 1-[2-(2,5-dimethoxyphenyl)ethyl]-4-(3-phenylpropyl)-piperazine | 3,9 | 369 |
| 39 | 732964-55-5 | 1-[3-(3,4-dimethoxyphenyl)propyl]-4-(4-phenylbutyl)-piperazine | 4,8 | 397 |
| 40 | 745775-91-1 | 4-[3-[4-[2-(3,4-dimethoxyphenyl)ethyl]-1-piperazinyl]propyl]-phenol | 3,0 | 384 |
| 41 | 759433-88-0 | 1-[3-(3,4-dimethoxyphenyl)propyl]-4-(3-phenylpropyl)-piperazine | 4,4 | 382 |
| 42 | nouveau | 1-[4-(3-phenylpropyl)-piperazine-1-yl] octan-3-one | 4,2 | 330 |
| 43 | nouveau | 1-[4-(3-phenylpropyl)piperazine-1-yl] octan-3-ol | 3,8 | 332 |
| 44 | nouveau | 1-[2-(benzyloxy)ethyl]-4-(3-phenylpropyl) piperazine | 3,8 | 338 |

Les dérivés de pipérazine selon l'invention peuvent notamment être préparés selon le schéma réactionnel suivant : 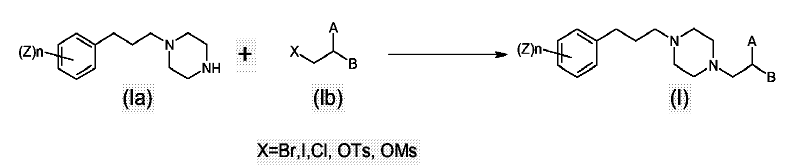 par réaction de la 1-(3-phenylpropyl)-pipérazine (la) (1 éq.) préalablement solubilisée dans un solvant organique polaire (0,5 à 1 M) tel que méthanol, éthanol, acétonitrile, DMF, DMSO, éventuellement additionné d'une base minérale (1 à 5 éq.) tel que NaHCO3, Na2CO3, NaOH, KOH, avec le réactif (Ib) (1 éq.). Le mélange est agité pendant une à dix heures, éventuellement chauffé jusqu'au reflux. La réaction est ensuite traitée et le produit purifié par des méthodes classiques.
Le réactif (la) peut être préparé en faisant régir 1 équivalent de pipérazine avec 1 équivalent de 1-cloro-3-aryle-propyle dans un solvant polaire comme le diméthylformamide, selon le procédé décrit dans J. Med. Chem, 1998, 25, 4950.

En variante, on peut remplacer le réactif (Ib) par le réactif (Ic) :

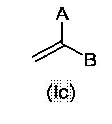

Les dérivés de pipérazine selon l'invention peuvent également être préparés comme décrit par Zikolova, S. Bul. Trudova 1978, 10, 33-46 ou par Matecka, D. et al. J. Med. Chem. 1996, 32(24), 4704-4716.

Certains dérivés de pipérazine sont décrits dans l'art antérieur, notamment dans les documents suivants :
Les composés 2 et 14 sont décrits dans la demande WO 2004/092123.
Le composé 3 est décrit dans les documents FR-A-2374318, US 5177077.
Le composé 4 est décrit dans la demande FR-A-2374322.
Le composé 5 est décrit dans la demande JP-A-2005-239594.
Les composés 17 et 18 sont décrits dans le brevet US 4616086.
Le composé 19 est décrit dans les documents WO 2001/00604, WO 2002/030422, US 6492369, US 5736546.
Le composé 21 est décrit dans l'article Rische et al, « One-pot synthesis of secondary and tertiary amines by carbonylative hydroaminomethylation of alkenes catalysed by di(µ-chloro)bis(η4-1,5-cyclooctadiene)dirhodium », Synthesis (1997), (11), 1331-1337.
Les composés 22, 23, 27, 28 sont décrits dans la demande US 2003-171347.
Les composés 22 à 30 sont décrits dans l'article Zhang et al, « Characterization of novel N,N'-disubstituted piperazines as sigma receptor ligands », Journal of medicinal chemistry (1998), 41(25), 4950-4957.
Le composé 31 est décrit dans l'article Benedetti et al, « GBR compounds and mepyramines as cocaine abuse therapeutics : chemometric studies on selectibvity using grid independent descriptors », Journal of medicinal chemistry (2002), 45(8), 1577-1584.
Les composés 20 et 32 à 35 sont décrits dans l'article Kawamura et al, « Synthesis and evaluation of 11C- and 18F- labeled 1-[2-(4-alkoxy-3-methoxyphenyl)ethyl]-4-(3-phenylpropyl)piperazines as sigma receptor ligands for positron émission tomography studies », Nuclear medicine and biology (2003), 30(3), 273-284.

Certains des dérivés de pipérazine selon l'invention sont nouveaux.

L'invention a donc également pour objet de nouveaux dérivés de pipérazine de formule (IIa) ou (IIb) : 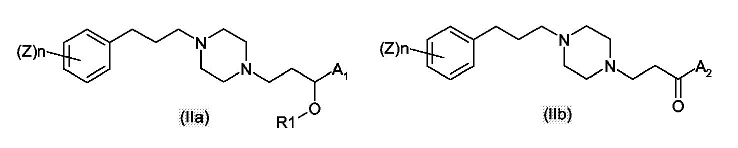 dans lesquelles :
chacun des groupes Z désigne indépendamment un atome de fluor, un radical alkyle linéaire en C₁-C₆ ou ramifié en C₃-C₆, un radical alkényle linéaire en C₂-C₆ ou ramifié en C₃-C₆, un radical cycloalkyle en C₃-C₆, un groupe CF₃, un groupe OR₁, un groupe NR₁R₂, un groupe COR₁, un groupe COOR₁, un groupe CONR₁R₂ ou un groupe N(R₁)COR₂ ;
n va de 0 à 5 ;
R₁ et R₂ désignent indépendamment un atome d'hydrogène, un radical alkyle en C₁-C₆ linéaire ou en C₃-C₆ ramifié, éventuellement substitué par un radical phényle, un radical alkényle en C₂-C₆ linéaire ou en C₃-C₆ ramifié, un radical cycloalkyle en C₃-C₆ ou un radical phényle ; les groupes R₁, respectivement R₂, pouvant être identiques ou différents les uns des autres ; et
A₁ et A₂ désignent indépendamment un radical alkyle linéaire en C₁-C₁₉ ou ramifié en C₃-C₁₉, un radical alkényle linéaire en C₂-C₁₉ ou ramifié en C₃-C₁₉ ou un radical cycloalkyle en C₃-C₁₉, lesdits radicaux étant éventuellement substitués par au moins un groupe choisi parmi : un radical oxo, un groupe OR₁, un groupe NR₁R₂, un groupe COR₁, un groupe COOR₁, un groupe CONR₁R₂, un groupe N(R₁)COR₂, ou un radical phényle éventuellement substitué par un à cinq groupes Z tels que définis précédemment.

De préférence, n est égal à 0.

En outre, on préfère que R₁ désigne un atome d'hydrogène ou un radical alkyle en C₁₋C₆ linéaire ou en C₃-C₆ ramifié, plus préférentiellement un atome d'hydrogène.

En outre, A₁ et A₂ désignent avantageusement un radical alkyle linéaire en C₁-C₆ ou ramifié en C₃-C₆ qui n'est de préférence pas substitué.

De préférence, pour les composés de formule (IIa) :
A₁ désigne un radical alkyle linéaire en C₁-C₆ , R₁ désigne un atome d'hydrogène et n =0
   ou
A₁ désigne un atome d'hydrogène, R₁ désigne un radical alkyle linéaire en C₁-C₆ éventuellement substitué par un radical phényle et n = 0.

De préférence, pour les composés de formule (IIb), A₂ désigne un radical alkyle linéaire en C₁-C₆ et n = 0.

La quantité de dérivé de pipérazine utilisable selon l'invention est bien entendu fonction de l'effet recherché et peut donc varier dans une large mesure.

Pour donner un ordre de grandeur, on peut utiliser ces dérivés en une quantité représentant de 0,01% à 10% du poids total de la composition, préférentiellement en une quantité représentant de 0,05% à 5% du poids total de la composition, plus préférentiellement en une quantité représentant de 0,1% à 2% du poids total de la composition.

Cette composition peut se présenter sous toutes les formes galéniques normalement utilisées dans le domaine cosmétique, et elle peut être notamment sous forme d'une solution éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des composés selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et co-émulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants / épaississants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants / épaississants lipophiles, on peut citer les argiles modifiées comme les bentones, les sels métalliques d'acides gras, et la silice hydrophobe.

Comme actifs, il sera avantageux d'introduire dans la composition utilisée selon l'invention au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les autres agents myorelaxants et/ou dermo-décontractants ; les agents tenseurs ; les agents anti-pollution et/ou anti-radicalaire ; les agents agissant sur la microcirculation ; les agents agissant sur le métabolisme énergétique des cellules ; et leurs mélanges.

Des exemples de tels composés additionnels sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les protéines végétales et leurs hydrolysats, ainsi que les phytohormones ; les extraits marins tels que les extraits d'algues ; les extraits bactériens ; les sapogénines telles que la diosgénine et les extraits de Dioscorées, notamment de Wild Yam, contenant de la diosgénine ; les céramides ; les hydroxyacides ; les hydroxyacides, tels que l'acide salicylique et l'acide n-octanoyl-5-salicylique ; le resvératrol ; les oligopeptides et pseudodipeptides et leurs dérivés acylés ; les sels de manganèse et de magnésium, en particulier les gluconates ; et leurs mélanges.

Comme indiqué précédemment, la composition selon l'invention peut également renfermer des agents photo-protecteurs actifs dans l'UVA et/ou l'UVB, sous forme de composés organiques ou minéraux, ces derniers étant éventuellement enrobés pour les rendre hydrophobes.

Les agents photo-protecteurs organiques peuvent notamment être choisis parmi : les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-3 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane.

Les agents photo-protecteurs inorganiques sont de préférence constitués d'oxyde de zinc et/ou de dioxyde de titane, de préférence de taille nanométrique, éventuellement enrobés d'alumine et/ou d'acide stéarique.

L'invention sera maintenant illustrée par les exemples non limitatifs suivants. Dans ces exemples, les quantités sont indiquées en pourcentage pondéral.

### EXEMPLES

### Exemple 1 : Synthèse de la 1,4-bis(3-phenylpropyl) piperazine

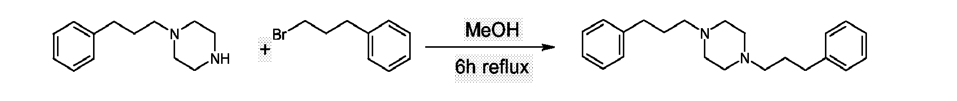

Dans un ballon, on a solubilisé 1g (4.9 mmoles) de 1-(3-phenylpropyl)-piperazine dans 20ml de méthanol. Puis on a ajouté 0.85ml (5.4 mmole) de 1-bromo-3-phenyl-propane et chauffé 6h au reflux. Le milieu réactionnel a ensuite été refroidi à température ambiante, et dilué avec de l'acétate d'éthyle et de l'eau. La phase aqueuse a été extraite avec de l'acétate d'éthyle. Les phases organiques ont ensuite été rassemblées, lavées avec une solution saturée de NaCI, séchées, et concentrées sous vide. Le résidu brut a été purifié sur colonne de silice (EtOAc/Heptane), pour donner 0.65g (rendement = 41%) de 1,4-bis(3-phenylpropyl) piperazine sous forme d'une huile.

Les analyses par spectrométrie de masse et par RMN étaient conformes à la structure attendue.

### Exemple 2 : Synthèse de la 1-[(2E)-3-phenylprop-2-enyl]-4-(3-phenylpropyl) piperazine

Ce composé peut être obtenu en suivant le protocole de l'exemple 2, et en utilisant le bromure de cinnamoyle à la place du 1-bromo-3-phenyl-propane.
Ce composé est notamment commercialisé par la société Chembridge (réf. 5428132).

### Exemple 3 : Synthèse de la 1-[4-(3-phenylpropyl)-piperazine-1-yl] octan-3-one

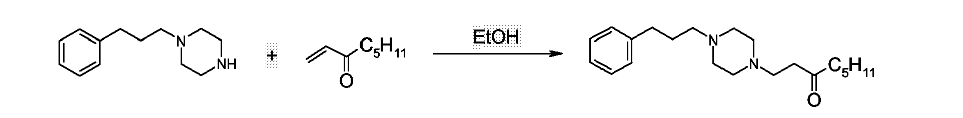

Dans un ballon, on solubilise 1g (4.9 mmoles) de 1-(3-phenylpropyl)-piperazine dans 10ml d'éthanol. On ajoute 0.620g de 1-octène-3-one (4.9 mmole) et on laisse réagir 15h à température ambiante. Le milieu réactionnel est dilué avec de l'acétate d'éthyle et de l'eau. La phase aqueuse est extraite avec de l'acétate d'éthyle. Les phases organiques sont ensuite rassemblées, lavées avec une solution saturée de NaCl, séchées, et concentrées sous vide. Le résidu brut est purifié sur colonne de silice (EtOAc/Heptane), pour donner 1.3g (rendement = 81%) de 1-[4-(3-phenylpropyl)-piperazine-1-yl] octan-3-one sous forme d'une huile incolore.

Les analyses par spectrométrie de masse et par RMN sont conformes à la structure attendue.

### Exemple 4 : Synthèse du 1-[4-(3-phenylpropyl)piperazine-1-yl] octan-3-ol

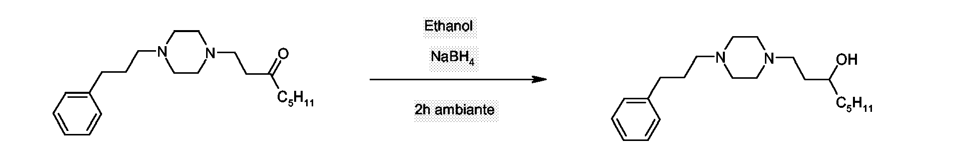

Dans un ballon, on solubilise 1.62g (4.9 mmoles) de 1-[4-(3-phenylpropyl)-piperazine-1-yl] octan-3-one dans10ml d'éthanol. On ajoute 0.278g de borohydrure de sodium en granules (7.35 mmoles) et on laisse réagir 2h à température ambiante. On ajoute ensuite de l'acétone pour éliminer le réducteur en excès. Le milieu réactionnel est dilué avec de l'acétate d'éthyle et de l'eau. La phase aqueuse est extraite avec de l'acétate d'éthyle. Les phases organiques sont ensuite rassemblées, lavées avec une solution saturée de NaCI, séchées, et concentrées sous vide. Le résidu brut est purifié sur colonne de silice (EtOAc/Heptane), pour donner 1.1g (rendement = 67%) du 1-[4-(3-phenylpropyl)piperazine-1-yl] octan-3-ol sous forme d'une huile incolore.

Les analyses par spectrométrie de masse et par RMN sont conformes à la structure attendue.

### Exemple 5 : Synthèse de la 1-[2-(benzyloxy)ethyl]-4-(3-phenylpropyl)-piperazine

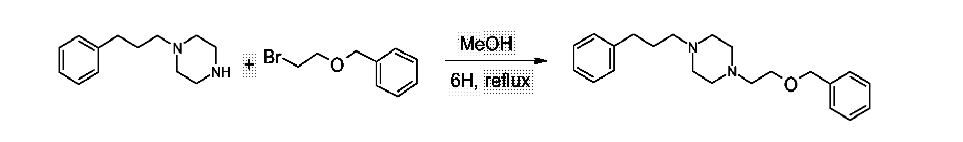

Dans un ballon, on a solubilisé 1g (4.9 mmoles) de 1-(3-phenylpropyl)-piperazine dans 20ml de méthanol. On a ajouté 0.8ml de benzyl-2-bromo-ethylether (4.9 mmole). On a ensuite chauffé le mélange à reflux pendant 6h. Le milieu réactionnel a été refroidi à température ambiante et dilué avec de l'acétate d'éthyle et de l'eau. La phase aqueuse a été extraite avec de l'acétate d'éthyle. Les phases organiques ont ensuite été rassemblées, lavées avec une solution saturée de NaCI, séchées, et concentrées sous vide. Le résidu brut a été purifié sur colonne de silice (EtOAc/Heptane), pour donner 1.3g (rendement = 79%) de 1-[2-(benzyloxy)ethyl]-4-(3-phenylpropyl)-piperazine sous forme d'une huile jaune.

Les analyses par spectrométrie de masse et par RMN étaient conformes à la structure attendue.

### Exemple 6 : Synthèse de la 1-heptyl-4-(3-phenylpropyl)piperazine

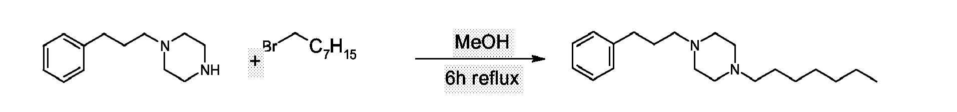

Dans un ballon, on a solubilisé 1g (4.9 mmoles) de 1-(3-phenylpropyl)-piperazine dans 25ml de méthanol. On a ajouté 1.17ml (4.9 mmole) de 1-bromoheptane et chauffé 6h au reflux. Le milieu réactionnel a ensuite été refroidi à température ambiante, et dilué avec de l'acétate d'éthyle et de l'eau. La phase aqueuse a été extraite avec de l'acétate d'éthyle. Les phases organiques ont ensuite été rassemblées, lavées avec une solution saturée de NaCl, séchées, et concentrées sous vide. Le résidu brut a été purifié sur colonne de silice (EtOAc/Heptane), pour donner 0.9g (rendement = 44%) de 1-heptyl-4-(3-phenylpropyl)piperazine sous forme d'une huile.

Les analyses par spectrométrie de masse et par RMN étaient conformes à la structure attendue.

### Exemple 7 : Mise en évidence de l'effet myorelaxant des dérivés selon l'invention

### Exemple 7A : activité en test de co-culture nerf-muscle

Les composés des Exemples 1 et 2 ont été testés sur un modèle de co-culture nerfs-muscles qui permet de recréer un arc moteur en innervant des cellules musculaires striées humaines avec des explants de moelle épinière et de ganglions rachidiens d'embryons de rat.

Ce test est prédictif d'un effet anti-rides, comme cela a été mis en évidence par la Demanderesse dans le cas du diazépam, qui inhibait les contractions des fibres musculaires dans ce modèle et dont l'activité anti-rides a été démontrée in vivo.

### a) Protocole

Des cellules musculaires humaines, issues de prélèvements de muscles striés de donneur sain, sont ensemencées dans des puits de 1,8 cm² de section (boîtes de culture de 24 puits). Après 10 jours de culture, ces cellules forment une monocouche et fusionnent. A ce stade, des explants de moelle épinière d'embryons de rat de 13 jours contenant les ganglions rachidiens sont déposées sur la culture.

La croissance des neurites est visible en dehors de l'explant de moelle épinière après un jour de culture. Les premières contractions des fibres musculaires sont observées après 5 à 6 jours de co-culture et après 3 semaines, toutes les fibres musculaires au voisinage des explants se contractent.

Le co-cultures sont utilisées après 21 jours, quand les fibres musculaires sont striées et possèdent des jonctions neuromusculaires différenciées matures.

Une fibre musculaire ayant des contractions régulières (au moins 60 contractions par minute) est alors sélectionnée dans trois puits de culture différents et le nombre de contractions est comptabilisé sur 30 secondes. Les composés testés, dilués dans le DMSO, sont ensuite incubés pendant 60 secondes dans ces puits, à la concentration de 10 µM pour le composé de l'Exemple 1 et de 10 et 100 µM pour le composé de l'Exemple 2. A la fin de l'incubation, le nombre de contractions est à nouveau comptabilisé sur 30 secondes. Le test est réalisé en triplicata.

### b) Résultats

| Composé | Concentration | % d'inhibition des contractions |
|---|---|---|
| Exemple 1 | 10 µM | 65% |
| | 100 µM | 100% |
| Exemple 2* | 10 µM | 50 % |
| | 100 µM | 100% |

| | | |
|---|---|---|
| * Commercialisé par Chembridge (réf. :5428132) | | |

Les composés selon l'invention inhibent donc les contractions des muscles striés et peuvent ainsi être utilisés pour détendre les traits du visage et lisser les rides d'expression.

### Exemple 78 : activité en test de binding sur canaux calciques de type L

### a) Protocole

On a évalué la capacité des composés des Exemples 1, 4 et 5 (à 1 µM dans le DMSO) à inhiber par compétition la fixation d'agonistes de canaux calciques de type L.

Les études sont réalisées à partir d'homogénats de cortex cérébral de rat (membranes isolées présentant à leur surface des canaux calciques de type L) selon la méthode décrite par Reynolds I.J. et al., 1986, J. Pharmacol. Exp. Ther., 237, p.731.

Les conditions expérimentales sont les suivantes :

| Test | Ligand | Conc. | Non spécifique | Incubation | Détection |
|---|---|---|---|---|---|
| Canal Ca ²⁺ (L, site verapamil) | [³H](-) D 888 | 0.5 nM | D 600 (10 µM) | 60min./22°C | Comptage scintillation |

D888 qui est le [³H](-) desmethoxyverapamil sert de ligand spécifique radiomarqué et D600 qui est le chlorhydrate de (±)méthoxyvérapamil sert de molécule de référence.

La liaison spécifique d'un ligand (D888 marqué) aux récepteurs (canaux calciques de type L, site vérapamil) est définie comme la différence entre la liaison totale et la liaison non spécifique déterminée en présence d'un excès de ligand froid (non radioactif). Les résultats sont exprimés en pourcentage d'inhibition de la liaison spécifique du témoin en présence du composé testé.

### b) Résultats

| COMPOSE | % inhibition de la liaison |
|---|---|
| Exemple 1 | 30% |
| Exemple 4 | 22% |
| Exemple 5 | 16% |

Les composés selon l'invention inhibent donc la liaison spécifique du témoin aux canaux calciques.

En outre, un test similaire a montré que le composé de l'Exemple 2 inhibait de 50% la liaison spécifique du témoin aux canaux calciques, à une concentration de 50 µM.

De ce test et de l'enseignement de la demande EP-1 053 745, on en déduit que ces composés présentent une forte probabilité d'avoir un effet bénéfique sur les rides et en particulier les rides d'expression.

### Exemple 8 : Composition cosmétique

Cette composition est préparée de manière classique pour l'homme du métier. Les quantités données dans cet exemple sont indiquées en pourcentages pondéraux.

| | |
|---|---|
| Composé de l'Exemple 2 | 0,10 % |
| Acide stéarique | 3,00 % |
| Mélange de mono-stéarate de glycéryle et stéarate de polyéthylène glycol (100 OE) | 2,50 % |
| Stéarate de polyéthylène glycol (20 OE) | 1,00 % |
| Cyclopentadiméthylsiloxane | 10,00 % |
| Charges | 3,00 % |
| Huiles végétales | 7,00 % |
| Huiles synthétiques | 6,00 % |
| Conservateurs | 1,20 % |
| Diméthylsiloxane oxyéthyléné (16 OE) à extrémités méthoxy | 1,00 % |
| Gomme de silicone | 0,20 % |
| Copolymère acrylique en émulsion inverse (Simulgel 600 de SEPPIC) | 1,70 % |
| Alcool stéarylique | 1,00 % |
| Eau | qsp 100 % |

Cette crème est destinée à être appliquée sur le visage et le front pour détendre les traits et décontracter la peau du visage.

## Revendications

1. Composition comprenant un milieu physiologiquement acceptable adapté à une application topique sur la peau, qui contient au moins un dérivé de pipérazine choisi parmi : les composés de formule (I) : 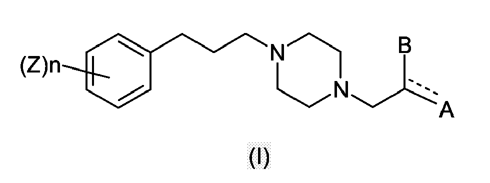 dans laquelle :
chacun des groupes Z désigne indépendamment un atome de fluor, un radical alkyle linéaire en C₁-C₆ ou ramifié en C₃-C₆, un radical alkényle linéaire en C₂-C₆ ou ramifié en C₃-C₆, un radical cycloalkyle en C₃-C₆, un groupe CF₃, un groupe OR₁, un groupe NR₁R₂, un groupe COR₁, un groupe COOR₁, un groupe CONR₁R₂ ou un groupe NR₁COR₂ ;
n va de 0 à 5 ;
A désigne :
- un atome d'hydrogène,
- un radical alkyle linéaire en C₁-C₂₀ ou ramifié en C₃-C₂₀ ,
- un radical alkényle en C₂-C₂₀ linéaire ou en C₃-C₂₀ ramifié,
- un radical cycloalkyle en C₃-C₂₀, lesdits radicaux étant éventuellement substitués par au moins un groupe choisi parmi : un radical oxo, un groupe OR₁, un groupe NR₁R₂, un groupe COR₁, un groupe COOR₁, un groupe CONR₁R₂, un groupe NR₁COR₂, ou un radical phényle éventuellement substitué par un à cinq groupes Z tels que définis ci-dessus,
- un radical phényle éventuellement substitué par un à cinq groupes Z tels que définis ci-dessus
B désigne un atome d'hydrogène, un radical alkyle linéaire en C₁-C₆ ou ramifié en C₃₋C₆, un radical alkényle linéaire en C₂-C₆ ou ramifié en C₃-C₆, un radical cycloalkyle en C₃-C₆, un groupe OR₁, un groupe NR₁R₂, un groupe COR₁, un groupe COOR₁, un groupe CONR₁R₂ ou un groupe NR₁COR₂ ;
où R₁ et R₂ désignent indépendamment un atome d'hydrogène, un radical alkyle linéaire en C₁-C₆ ou ramifié en C₃-C₆ éventuellement substitué par un radical phényle, un radical alkényle linéaire en C₂-C₆ ou ramifié en C₃-C₆, un radical cycloalkyle en C₃-C₆ ou un radical phényle ; les groupes R₁, respectivement R₂, pouvant être identiques ou différents les uns des autres ;
les pointillés désignent une double liaison potentielle, étant entendu que A est distinct d'un atome d'hydrogène ou d'un radical phényle lorsque cette double liaison est présente,
et leurs sels, isomères optiques et solvates.

2. Composition selon la revendication 1, **caractérisée en ce que** le dérivé de pipérazine est tel que : lorsque B est un atome d'hydrogène, alors A est distinct d'un groupe phényle éventuellement substitué.

3. Composition selon la revendication 1 ou 2, **caractérisée en ce que** chacun des groupes Z désigne indépendamment un groupe choisi parmi : un atome de fluor, un groupe OR₁, un groupe NR₁R₂, un groupe NR₁COR₂, un groupe COOR₁ ou un groupe CF₃.

4. Composition selon la revendication 3, **caractérisée en ce que** Z désigne un groupe OR₁ ou COOR₁.

5. Composition selon la revendication 3 ou 4, **caractérisée en ce que** R₁ et R₂ désignent indépendamment : un atome d'hydrogène ou un radical alkyle en C₁-C₆ linéaire ou ramifié, de préférence un radical méthyle ou éthyle.

6. Composition selon la revendication 1 ou 2, **caractérisée en ce que** n = 0.

7. Composition selon l'une quelconque des revendications 1 à 6, **caractérisée en ce que** A désigne :
- un atome d'hydrogène,
- un radical alkyle linéaire en C₁-C₈ ou ramifié en C₃-C₈,
- un radical alkényle linéaire en C₂-C₈ ou ramifié en C₃-C₈,
- un radical cycloalkyle en C₃-C₈, lesdits radicaux étant éventuellement substitués par au moins un groupe choisi parmi : un radical oxo, un groupe OR₁, un groupe NR₁R₂, ou un radical phényle éventuellement substitué par un à cinq groupes Z tels que définis dans la formule (I), ou
- un radical phényle éventuellement substitué par un à cinq groupes Z tels que définis dans la formule (I).

8. Composition selon la revendication 7, **caractérisée en ce que** A désigne un atome d'hydrogène ou un radical alkyle linéaire en C₁-C₆ ou ramifié en C₃-C₆, éventuellement substitué par au moins un groupe choisi parmi : un radical oxo, un groupe OR₁, ou un radical phényle, où R₁ désigne de préférence un atome d'hydrogène ou peut désigner un radical alkyle linéaire en C₁-C₆ ou ramifié en C₃-C₆ tel qu'un radical méthyle.

9. Composition selon l'une quelconque des revendications 1 à 8, **caractérisée en ce que** B désigne un atome d'hydrogène ; un radical alkyle en C₁-C₆ linéaire ou en C₃-C₆ ramifié ; un groupe NR₁R₂ ; un groupe COR₁ ; ou un groupe OR1, où R₁ désigne un atome d'hydrogène, un radical alkyle en C₁-C₆, linéaire ou en C₃-C₆ ramifié, éventuellement substitué par un radical phényle.

10. Composition selon la revendication 9, **caractérisée en ce que** B désigne un atome d'hydrogène, un groupe OR₁ ou un groupe COR₁ où R₁ désigne un radical alkyle linéaire en C₁-C₆ linéaire ou ramifié en C₃-C₆, éventuellement substitué par un radical phényle.

11. Composition selon l'une quelconque des revendications précédentes, **caractérisée en ce que** :
- A désigne un radical alkyle linéaire en C₁-C₈ éventuellement substitué par un radical phényle, B désigne un atome d'hydrogène ou un groupe hydroxyle, et n = 0 ;
ou
- A désigne un atome d'hydrogène, B désigne -OR₁ ou -COR₁ avec R₁ désignant un radical alkyle linéaire en C₁-C₆ éventuellement substitué par un radical phényle et n = 0.

12. Utilisation cosmétique de la composition selon l'une quelconque des revendications 1 à 11 pour le soin de la peau, en particulier pour le soin de la peau ridée et/ou pour lisser les rides, détendre les traits et/ou décontracter la peau.

13. Utilisation cosmétique d'au moins un dérivé de pipérazine tel que défini dans l'une quelconque des revendications 1 à 11, comme agent pour atténuer les rides et/ou décontracter la peau et/ou détendre les traits.

14. Procédé de traitement cosmétique d'une peau ridée, en particulier la peau du visage et/ou du front, comprenant l'application topique sur ladite peau d'une composition telle que définie dans l'une quelconque des revendications 1 à 11.

15. Procédé selon la revendication précédente, **caractérisé en ce que** la composition est appliquée sur les zones du visage et/ou du front marquées par des rides d'expression, et/ou sur les personnes présentant des rides d'expression.

16. Dérivés de pipérazine de formule (IIa) ou (IIb) : 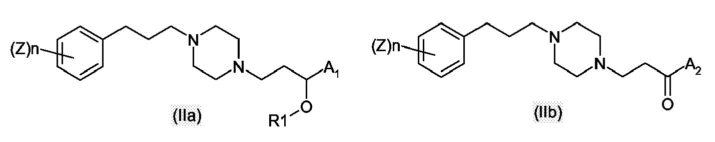 dans lesquelles :
chacun des groupes Z désigne indépendamment un atome de fluor, un radical alkyle linéaire en C₁-C₆ ou ramifié en C₃-C₆, un radical alkényle linéaire en C₂-C₆ ou ramifié en C₃-C₆, un radical cycloalkyle en C₃-C₆, un groupe CF₃, un groupe OR₁, un groupe NR₁R₂, un groupe COR₁, un groupe COOR₁, un groupe CONR₁R₂ ou un groupe NR₁COR₂ ;
n va de 0 à 5 ;
R₁ et R₂ désignent indépendamment un atome d'hydrogène, un radical alkyle en C₁-C₆ linéaire ou en C₃-C₆ ramifié, éventuellement substitué par un radical phényle, un radical alkényle en C₂-C₆ linéaire ou en C₃-C₆ ramifié, un radical cycloalkyle en C₃-C₆ ou un radical phényle ; les groupes R₁, respectivement R₂, pouvant être identiques ou différents les uns des autres ; et
A₁ et A₂ désignent indépendamment un radical alkyle linéaire en C₁-C₁₉ ou ramifié en C₃-C₁₉, un radical alkényle linéaire en C₂-C₁₉ ou ramifié en C₃-C₁₉ ou un radical cycloalkyle en C₃-C₁₉, lesdits radicaux étant éventuellement substitués par au moins un groupe choisi parmi : un radical oxo, un groupe OR₁, un groupe NR₁R₂, un groupe COR₁, un groupe COOR₁, un groupe CONR₁R₂, un groupe NR₁COR₂, ou un radical phényle éventuellement substitué par un à cinq groupes Z tels que définis précédemment.

17. Dérivés de pipérazine selon la revendication précédente, **caractérisés en ce que** n est égal à 0.

18. Dérivés de pipérazine selon la revendication 16 ou 17, **caractérisés en ce que** R₁ désigne un atome d'hydrogène ou un radical alkyle linéaire en C₁-C₆ ou ramifié en en C₃-C₆.

19. Dérivés de pipérazine selon la revendication 18, **caractérisés en ce que** R₁ désigne un atome d'hydrogène.

20. Dérivés de pipérazine selon l'une quelconque des revendications 16 à 19, **caractérisés en ce que** A₁ et A₂ désignent avantageusement un radical alkyle linéaire en C₁-C₆ ou ramifié en C₃-C₆ non substitué.

21. Dérivés de pipérazine de formule (IIa) selon l'une quelconque des revendications 16 à 19, **caractérisés en ce que** :
- A₁ désigne un radical alkyle linéaire en C₁-C₆ , R₁ désigne un atome d'hydrogène et n=0
ou
- A₁ désigne un atome d'hydrogène, R₁ désigne un radical alkyle linéaire en C₁-C₆ éventuellement substitué par un radical phényle et n = 0.

22. Dérivés de pipérazine de formule (IIa) selon l'une quelconque des revendications 16 à 19, **caractérisés en ce que** A₂ désigne un radical alkyle linéaire en C₁-C₆ et n = 0.
